# FASCICULE DE BREVET EUROPEEN

(11) **EP 2 785 857 B1**
(45) Date de publication et mention de la délivrance du brevet: **04.05.2016**
(21) Numéro de dépôt: 12791767.2
(22) Date de dépôt: 28.11.2012
(51) Int. Cl.: C12Q 1/04, C12Q 1/10

(54) **PROCEDE DE DETECTION DE BACTERIES YERSINIA ENTEROCOLITICA PATHOGENES**
VERFAHREN ZUM NACHWEIS PATHOGENER YERSINA ENTEROCOLITICA-BAKTERIEN
METHOD FOR DETECTING PATHOGENIC YERSINIA ENTEROCOLITICA BACTERIA

(30) Priorité: 28.11.2011 FR 1160876
(43) Date de publication de la demande: 08.10.2014
(73) Titulaire: Rambach, Alain, 75006 Paris (FR)
(72) Inventeur: Rambach, Alain, 75006 Paris (FR)
(74) Mandataire: Regimbeau
(86) Numéro de dépôt international: PCT/EP2012/073802
(87) Numéro de publication internationale: WO 2013/079513

(56) Documents cités:
- FR-A1- 2 767 535
- US-A- 4 952 497
- WEAGANT ET AL: "A new chromogenic agar medium for detection of potentially virulent Yersinia enterocolitica", JOURNAL OF MICROBIOLOGICAL METHODS, ELSEVIER, AMSTERDAM, NL, vol. 72, no. 2, 4 décembre 2007 (2007-12-04), pages 185-190, XP022420008, ISSN: 0167-7012, DOI: 10.1016/J.MIMET.2007.11.019
- DUDLEY M V ET AL: "Medium for isolation of Yersinia enterocolitica.", JOURNAL OF CLINICAL MICROBIOLOGY AUG 1979 LNKD- PUBMED:511986, vol. 10, no. 2, août 1979 (1979-08), pages 180-183, XP002683368, ISSN: 0095-1137
- FUKUSHIMA H: "New selective agar medium for isolation of virulent Yersinia enterocolitica.", JOURNAL OF CLINICAL MICROBIOLOGY JUN 1987 LNKD- PUBMED:3597750, vol. 25, no. 6, juin 1987 (1987-06), pages 1068-1073, XP002683369, ISSN: 0095-1137

## Description

La présente invention concerne un milieu de culture pour la détection de bactéries *Yersinia enterocolitica* pathogènes comprenant au moins un agent chromogène substrat d'une acétyl-glucosaminidase, un procédé de détection utilisant un tel milieu et l'utilisation d'un tel milieu pour la détection de bactéries *Yersinia enterolitica* pathogènes.

Les *Yersinia* sont des entérobactéries regroupant actuellement trois espèces contenant des souches pathogènes pour l'animal et l'homme: *Yersinia pestis, Yersinia enterocolitica* et *Yersinia pseudotuberculosis,* et des espèces habituellement non pathogènes, *Yersinia frederiksenii*, *Yersinia kristensenii, Yersinia intermedia, Yersinia aldovae*, *Yersinia mollaretii, Yersinia bercovieri* et *Yersinia rodhei.*

*Yersinia enterocolitica* est responsable des entérocolites. Elle est rapportée, le plus souvent chez l'enfant et se traduit par des diarrhées, de la fièvre, des douleurs abdominales et parfois des vomissements.

La recherche des *Yersinia* a conduit au développement de nombreux milieux de culture. Ces milieux, généralement incubés à la température de 30°C, notamment pour conserver, exprimer et conserver le caractère pathogène, ont conduit à développer des méthodes pour caractériser des souches pathogènes, par exemple en repérant à la température plus élevée de 37°C, anormale pour cette espèce, une réduction de la croissance sur des milieux déficients en calcium et une coloration plus intense par le cristal violet.

Parmi les milieux d'isolement de l'art antérieur, on peut citer le milieu CIN agar (Schiemann DA, Synthesis of a selective agar medium for Yersinia enterocolitica, Can. J Microbiol., 1979) qui emploie pour détecter *Yersinia* le caractère mannitol révélé par un changement de pH et un indicateur coloré, le milieu CAL agar (Dudley et al., Medium for isolation of Yersinia enterocolitica, Journal of Clinical Biology, 1979) qui emploie le caractère cellobiose révélé par un changement de pH et un indicateur coloré. D'autre part, un milieu VYE agar qui emploie le caractère esculine pour distinguer les non pathogènes par un halo noir diffusant autour des colonies, a également été élaboré (Fukushima, New Selective Agar Medium for Isolation of Virulent Yersinia enterocolitica, Journal of Clinical Biology, 1987). Weagant a développé un milieu dérivé du CIN Agar en remplaçant le mannitol par du cellobiose et en ajoutant du X-glucoside (5-bromo-4-chloro-3-indoyl-béta-D-glucopyranoside) (Weagant, A new chromogenic agar medium for detection of potentially virulent Yersinia enterocolitica, Elsevier, 2008).

Garcia-Aguayo *et al*. ont développé en 1999 un milieu Xylose-Galactosidase au 5-bromo 6-chloro-3-indoxyl-beta-galactoside qui emploie le caractère beta-galactosidase (Garcia-Aguayo et al., Evaluation ofXylose-Galactosidase Medium, a new Plate for the Insolation of Salmonella, Shigella, Yersinia and Aeromonas Species, Eur J Clin Microbio Infect Dis., Volume 18, 1999).

De tels milieux ne permettent cependant pas d'obtenir une sensibilité suffisante et donnent également lieu à de faux positifs ce qui limite la fiabilité des résultats.

Il est donc important de disposer d'outils et de procédés de détection de ces bactéries qui combinent à la fois une bonne sensibilité et une bonne spécificité, et surtout une grande facilité d'usage de façon à pouvoir autant que possible simplifier les tests, les faire rapidement et en grand nombre, voire les automatiser, dans une perspective de contrôle de l'hygiène alimentaire et/ou hospitalière, tout en permettant de différencier rapidement différentes souches *Yersinia enterocolitica* pathogènes.

Il existe donc un réel besoin de disposer d'une technique de détection plus simple, plus spécifique, plus directe et moins coûteuse et permettant de différencier différentes souches de bactéries *Yersinia enterocolitica* pathogènes, tout en évitant de combiner plusieurs tests générant un délai supplémentaire pour l'obtention des résultats et augmentant le risque de contaminations parasites ou d'erreur ainsi que le risque de la diffusion de la bactérie.

Des milieux comprenant des indoxyl-beta-galactosides ont été testés. Une coloration des colonies de *Yersinia enterocolitica* a été observée, mais celle-ci apparaissait lentement. De plus, une coloration se développait également sur un très grand nombre de souches d'espèces non*-Yersinia*.

De manière surprenante et inattendue, l'Inventeur a montré que l'utilisation de substrats chromogènes de l'hexosaminidase, en particulier de l'acétyl-glucosaminidase, permet d'isoler rapidement les bactéries *Yersinia enterocolitica* pathogènes de façon sensible et spécifique. En particulier, lorsqu'il est mis en oeuvre sur un milieu solide gélosé, le procédé de détection développé par l'Inventeur est réalisable directement, par exemple à partir d'un échantillon alimentaire ou issu d'un patient, sans nécessiter d'étape d'isolement préalable des différentes souches présentes dans cet échantillon.

Un objet de la présente invention consiste donc en un procédé de détection directe de bactéries *Yersinia enterocolitica* pathogènes dans un échantillon biologique comprenant un milieu de culture pour la détection desdites bactéries comprenant:
- les nutriments nécessaires à la croissance desdites bactéries à détecter, et
- au moins un agent chromogène substrat d'une acétyl-glucosaminidase.

Par « milieu de culture », on entend un milieu permettant la croissance desdites bactéries à détecter. Ledit milieu de culture comprend en effet les nutriments nécessaires à la croissance desdites bactéries à détecter.

Par « bactéries *Yersinia enterocolitica* pathogènes » on entend des bactéries *Yersinia enterocolitica* présentant un biotype choisi dans le groupe constitué par les biotypes 1B, 2, 3, 4 et 5. En effet, le biotype 1A de *Yersinia enterocolitica* est fréquent et n'est pas reconnu comme pathogène pour l'homme et les animaux.

Par « nutriments nécessaires à la croissance desdites bactéries à détecter », on entend la composition d'un milieu de base nécessaire à ladite croissance. L'homme du métier connaît parfaitement la composition de tels milieux et est à même de l'adapter si nécessaire en fonction de la spécificité de certains microorganismes ou de contraintes qui pourraient être liées à certains cas de la présente invention (transparence du milieu par exemple). Ces nutriments sont notamment choisis dans le groupe comprenant du carbone, de l'azote, du soufre, du phosphore, des vitamines, des inducteurs de croissance, des hydrates de carbone, des sels (par exemple calcium, magnésium, manganèse, sodium, potassium), des complexes nutritifs (par exemple des acides aminés, du sang, du sérum, de l'albumine) ainsi que des peptones et des extraits de tissus animaux et végétaux.

Le milieu de culture utilisé dans le cadre de la présente invention peut être sous forme solide, semi-solide, liquide ou lyophilisée. De préférence, ledit milieu de culture est un milieu gélosé, lequel milieu de culture est, à titre d'exemple, à base d'agar. Parmi les présentations des milieux de culture utilisables, on peut ainsi citer les boîtes de Pétri sur lesquelles se développent des microorganismes.

Par « agent chromogène » ou « substrat chromogène », on entend un composé portant un chromophore libéré après une hydrolyse par une enzyme spécifique. Le chromophore ainsi libéré donne sa couleur aux colonies comprenant ladite enzyme. De préférence, ledit substrat chromophore est un chromophore précipitant.

Au sens de la présente invention, on entend par acétyl-glucosaminidase une enzyme capable de libérer par hydrolyse un résidu N-acétyl-D-glucosamine.

Le substrat chromogène est de préférence utilisé à une concentration comprise entre 0,01 et 0,5 g/l, préférentiellement entre 0,05 et 0,2 g/l. Une concentration particulièrement préférée se situe à 0,1 g/l environ.

De façon préférée, ledit chromophore est choisi dans le groupe constitué par les dérivés indoxyle, halogéno-indoxyle (bromo-indoxyle, chloro-indoxyle, fluoro-indoxyle, iodo-indoxyle, dichloro-indoxyle, chloro-bromo-indoxyle, tri-chloro-indoxyle), le méthyl-indoxyle, et hydroxy-quinoline. Des dérivés particulièrement préférés sont choisi dans le groupe constitué par les dérivés suivants : 6-chloro-indoxyle, 5-bromo-indoxyle, 3-bromo-indoxyle, 6-fluoro-indoxyle, 5-iodo-indoxyle, 4,6-dichloro-indoxyle, 6,7-dichloro-indoxyle, 5-bromo-4-chloro-indoxyle, 5-bromo-6-chloro-indoxyle, 4,6,7-trichloro-indoxyle, N-méthyl-indoxyle et 8-hydroxy-quinoline.

Avantageusement, ladite acétyl-glucosaminidase a pour substrat chromogène un indoxyl-beta-glucosaminide, de préférence choisi dans le groupe constitué par le 5-bromo-6-chloro-3-indoxyl-N-acétyl-beta-D-glucosaminide, le 6-chloro-3-indolyl-N-acétyl-beta-D-glucosaminide et le 6-fluoro-indoxyl-N-acétyl-beta-D-glucosaminide, de manière particulièrement préférée le 5-bromo-6-chloro-3-indoxyl-N-acétyl-beta-D-glucosaminide.

Alternativement, ladite acétyl-glucosaminidase a pour substrat chromogène un indolyl-beta-glucosaminide, de préférence le 5-bromo-4-chloro-3-indolyl-N-acétyl-beta-D-glucosaminide.

Dans un mode de réalisation particulièrement préféré, le milieu de culture utilisé dans le cadre de la présente invention comprend en outre un agent chromogène substrat de la beta-glucosidase, de préférence ledit agent chromogène libérant après hydrolyse un chromophore de couleur distincte du chromophore susceptible d'être libéré après hydrolyse de l'agent chromogène substrat d'une acétyl-glucosaminidase.

De préférence ledit agent chromogène substrat de la beta-glucosidase est le 5-Bromo-4-chloro-3-indolyl-beta-D-glucoside (également dénommé X-glucoside) lorsque l'agent chromogène substrat de l'acétyl-glucosaminidase du milieu selon l'invention est choisi dans le groupe constitué par : le 5-bromo-6-chloro-3-indoxyl-N-acétyl-beta-D-glucosaminide, le 6-chloro-3-indolyl-N-acétyl-beta-D-glucosaminide et le 6-fluoro-indoxyl-N-acétyl-beta-D-glucosaminide.

De préférence, ledit agent chromogène substrat de la beta-glucosidase est la 8-hydroxy-quinoline-beta-D-glucoside (également dénommé X-glucoside) lorsque l'agent chromogène substrat de l'acétyl glucosaminidase du milieu de culture utilisé dans le cadre de la présente invention est le 5-bromo-4-chloro-3-indolyl-N-acétyl-beta-D-glucosaminide.

Ledit agent chromogène substrat de la beta-glucosidase est de préférence utilisé à une concentration allant de 0,01 à 0,5 g/l, préférentiellement de 0,05 à 0,2 g/l. Une concentration particulièrement préférée se situe à 0,1 g/l environ.

Dans un mode de réalisation particulièrement préféré, le milieu de culture utilisé dans le cadre de la présente invention comprend du 5-bromo-6-chloro-3-indoxyl-N-acétyl-beta-D-glucosaminide et du 5-Bromo-4-chloro-3-indolyl-beta-D-glucoside.

La quantité efficace de chromogène(s) dans le milieu de culture utilisé dans le cadre de la présente invention pourra être simplement ajustée par l'homme du métier au regard de ses connaissances générales et des résultats décrits dans les exemples ci-après.

Les milieux de culture utilisé dans le cadre de la présente invention peuvent éventuellement contenir un ou plusieurs agents antimicrobiens, en particulier un ou plusieurs antibiotiques et/ou un ou plusieurs antifongiques.

Le ou lesdits agents antimicrobiens permettent de limiter la croissance de microorganismes autres que ledit au moins un microorganisme spécifique à détecter. La quantité efficace d'agent antimicrobien à utiliser peut être déterminée simplement par l'homme du métier au regard de ses connaissances générales.

Avantageusement, le milieu de culture utilisé dans le cadre de la présente invention comprend en outre de l'Irgasan.

Au sens de la présente invention, on entend par Irgasan un composé de formule : 5-chloro-2-(2,4-dichlorophenoxy)phenol (Numéro CAS: 3380-34-5).

De préférence, le milieu de culture utilisé dans le cadre de la présente invention comprend du 5-chloro-2-(2,4-dichlorophenoxy)phenol à une concentration comprise entre 0,0005 et 0,01 g/l, de préférence comprise entre 0,001 et 0,005 g/l, préférentiellement comprise entre 0,001 et 0,003 g/l, et encore préférentiellement d'environ 0,002 g/l.

Un objet selon la présente invention se rapporte donc à un procédé de détection directe de bactéries *Yersinia enterocolitica* pathogènes dans un échantillon biologique comprenant les étapes successives:
a) d'inoculation, avec ledit échantillon, d'un milieu de culture comprenant:
   - les nutriments nécessaires à la croissance des bactéries Yersinia enterocolitica pathogènes, et
   - au moins un agent chromogène substrat d'une acétyl-glucosaminidase
b) d'incubation dudit milieu de culture dans des conditions permettant la croissance des bactéries Yersinia enterocolitica pathogènes, et
c) de détection des colonies formées sur ledit milieu de culture correspondant aux bactéries Yersinia enterocolitica pathogènes.

Avantageusement, le procédé selon la présente invention comprend en outre une étape d) permettant de conclure à la présence ou non d'une souche particulière de bactéries en fonction de la couleur des colonies formées. Il s'agit par conséquent d'une étape d) d'identification de bactéries *Yersinia enterocolitica* pathogènes, dans ledit échantillon.

Par « échantillon biologique», on entend tout type de prélèvement microbiologique, tel que par exemple un prélèvement de matières alimentaires (produits laitiers, viande, etc.), un prélèvement de sol, un prélèvement sur un mammifère (peau, muqueuses, etc.), de préférence l'homme, ou l'un de ses dérivés comme une préculture issue d'un tel prélèvement.

Avantageusement, ledit échantillon biologique est un échantillon biologique liquide, comme de la salive, du sang ou de l'urine, un échantillon biologique solide, comme des fèces ou un produit alimentaire, ou encore un dérivé d'un échantillon biologique liquide ou solide tel qu'une pré-culture d'un tel échantillon biologique liquide ou solide.

Avantageusement encore, ledit échantillon biologique comprend différents microorganismes, lesquels peuvent appartenir à des espèces voire à des genres distincts. À titre d'exemple, ledit échantillon biologique comprend au moins deux microorganismes différents, de préférence d'au moins cinq microorganismes différents et, de manière particulièrement préférée, d'au moins dix microorganismes différents. Par «inoculation», on entend la mise en culture dudit milieu de culture par tout ou partie de l'échantillon biologique et incubation dudit milieu de culture inoculé. L'homme du métier adaptera les conditions d'incubation en fonction du milieu de culture, de l'échantillon biologique et du microorganisme spécifique à détecter en fonction de ses connaissances générales.

L'étape d'incubation peut être réalisée à une température comprise entre 0°C à 44°C, de préférence comprise entre 20°C à 43°C, préférentiellement comprise entre 28 et 32°C, et encore préférentiellement à 30°C.

De préférence, l'étape d'incubation est réalisée pendant une durée comprise entre 16 et 36 heures, de préférence entre 18 et 24 heures.

Cependant, en fonction des moyens dont il disposera, l'homme du métier pourra adapter la température et la durée de cette étape d'incubation au regard de ses connaissances générales.

Par « procédé de détection directe », on entend un procédé qui ne comprend pas d'étape préalable d'isolement des différentes souches bactériennes présentes dans l'échantillon, de préférence un procédé qui ne comprend pas d'étape préalable d'isolement de chacune des souches bactériennes présentes dans l'échantillon.

En effet, le procédé selon l'invention permet d'éviter l'étape d'isolement de colonies de bactéries candidates pouvant ensuite être soumises à un test plus précis de confirmation. Il s'applique donc à un échantillon brut susceptible de comprendre un mélange de bactéries.

De préférence, le procédé selon la présente invention ne comprend pas d'étape préalable d'isolement des différentes souches bactériennes présentes dans l'échantillon.

Par rapport aux procédés antérieurs, le procédé développé par l'Inventeur permet une détection directe et rapide des bactéries *Yersinia enterocolitica* pathogènes.

Un autre objet de la présente invention se rapporte à l'utilisation d'un milieu de culture tel que défini ci-dessus, pour la détection et la différenciation directes de bactéries *Yersinia enterocolitica* pathogènes.

Les exemples qui suivent sont fournis à titre d'illustration et ne sauraient limiter la portée de la présente invention.

### EXEMPLE

### Exemple 1 : Comparaison du milieu selon la présente invention avec un milieu CIN agar

Le milieu CIN agar de Becton Dickinson utilisé, comprend les éléments suivants en g/L : Peptone 17,0, Peptone de protéose 3,0, Extrait de levure 2,0, D-Mannitol 20,0, Désoxycholate de sodium 0,5, Cholate de sodium 0,5, Pyruvate de sodium 2,0, Sulfate de magnésium heptahydré 0,01, Chlorure de sodium 1,0, Rouge neutre 0,03, Cristal Violet 0,001, Irgasan 0,004, Gélose 13,5, Cefsulodine 0,015, Novobiocine 0,0025.

Le milieu selon la présente invention comprend les éléments suivants en g/L: Peptone 20 ; Sodium Chloride 5 ; Agar 15 ; 5-Bromo-4-chloro-3-indolyl-beta-D-glucoside 0,1 ; 5-bromo 6-chloro 3-indoxyl-N-acétyl-β-D-glucosaminide 0,1 ; Irgasan 0,002 ; Tween 800,5.

Diverses souches bactériennes sont isolées sur le milieu de l'invention et le milieu CIN Agar. Les boites sont incubées 24h à 30°C.

Les résultats obtenus sont présentés dans le tableau I suivant :

**Tableau I**

| | CIN Agar | | Milieu selon l'invention | |
|---|---|---|---|---|
| | Couleur | Détection | Couleur | Détection |
| | | | | |
| *Y.enterocolitica* AR5580 | ROUGE | + | MAUVE | + |
| *Y.enterocolitica* 2B154 non-pathogène | ROUGE | + | BLEU | - |
| *Y.enterocolitica* AR5685 non-pathogène | ROUGE | + | BLEU | - |
| | | | | |
| *Citrobacter* AR 3378 | ROUGE | + | BLEU | - |
| *Citrobacter* AR 3870 | ROUGE | + | BLEU | - |
| *Citrobacter* AR 3871 | ROUGE | + | BLEU | - |
| *Citrobacter* AR 3030 | ROUGE | + | BLEU | - |
| *Serratia* AR 5568 | ROUGE | + | BLEU | - |
| | | | | |
| *E. coli* AR 3741 | NC | - | NC | - |
| *Hafnia* AR 3862 | NC | - | NC | - |
| *Enterobacter* AR 3412 | NC | - | NC | - |
| | | | | |
| *Morganella* AR4080 | incolore | - | incolore | - |
| *Pseudomonas* AR5196 | incolore | - | incolore | - |

| | | | | |
|---|---|---|---|---|
| NC : Non croissance des bactéries. | | | | |

Le tableau I montre que les deux milieux permettent de détecter les *Yersinia enterocolitica* pathogènes mais que de nombreuses espèces détectées à l'aide du milieu traditionel CIN Agar, sont en réalité des faux positifs, c'est-à-dire soit des souches *Yersinia enterocolitica* non pathogènes ou des non-*Yersinia enterocolitica,* ce qui n'est pas le cas avec le milieu selon la présente invention.

De manière surprenante, un milieu semblable à celui de la présente invention, mais dans lequel l'indoxyl-béta-glucosaminide a été remplacée par l'indoxyl-béta-galactosaminide, autre substrat d'hexosaminidase, ne permet pas d'obtenir une coloration des souches *Yersinia enterocolitica* pathogènes. Ainsi, un tel milieu ne permet pas de différencier *Yersinia enterocolitica* pathogènes des autres bactéries, démontrant ainsi, de manière surprenante, que seul un substrat du type glucosaminidine permet de détecter les souches *Yersinia enterocolitica* pathogènes avec une bonne spécificité et une bonne sensibilité.

### Exemple 2 : Comparaison du caractère de l'invention et du caractère beta-galactosidase

Formules : en g/L
Milieu A selon l'invention : Peptone 20 ; Sodium Chloride 5 : Agar 15 ; 5-bromo-6-chloro-3-indoxyl-N-acétyl-β-D-glucosaminide 0,1.
Milieu B : Peptone 20 ; Sodium Chloride 5 : Agar 15 ; IPTG 0,04 ; 5-bromo 6-chloro 3-indoxyl-beta-galactoside 0,1.

Les boites sont incubées 24h à 30°C.

Les résultats obtenus sont présentés dans le tableau II suivant :

**Tableau II**

| | Milieu A | | Milieu B | |
|---|---|---|---|---|
| | Couleur | Détection | Couleur | Détection |
| | | | | |
| *Y. enterocolitica* AR5580 | MAUVE | + | MAUVE | + |
| | | | | |
| *Citrobacter* AR 3030 | incolore | - | MAUVE | + |
| *Klebsiella* K3875 | incolore | - | MAUVE | + |
| *E. coli* AR 3740 | incolore | - | MAUVE | + |
| *E. coli* ATCC25922 | incolore | - | MAUVE | + |

Les résultats présentés dans le tableau II montrent que, contrairement au milieu de l'invention, le milieu B ne permet pas d'éliminer des faux positifs.

Ainsi, le milieu de culture utilisé selon la présente invention présente une sensibilité et une spécificité supérieures, permettant ainsi une détection plus facile, en une seule étape de culture sans nécessité d'une étape préalable d'isolement ni étape ultérieure de différenciation. Ceci a pour conséquence de diminuer des coûts associés et également d'obtenir un rendement amélioré.

## Revendications

1. Procédé de détection directe de bactéries *Yersinia enterocolitica* pathogènes dans un échantillon comprenant les étapes successives:
a) d'inoculation, avec ledit échantillon, d'un milieu de culture comprenant:
- les nutriments nécessaires à la croissance des bactéries *Yersinia enterocolitica* pathogènes, et
- au moins un agent chromogène substrat d'une acétyl-glucosaminidase
b) d'incubation dudit milieu de culture dans des conditions permettant la croissance des bactéries *Yersinia enterocolitica* pathogènes, et
c) de détection des colonies formées sur ledit milieu de culture correspondant aux bactéries *Yersinia enterocolitica* pathogènes.

2. Utilisation d'un milieu de culture pour la détection et la différenciation directes de bactéries *Yersinia enterocolitica* pathogènes, **caractérisé en ce que** ledit milieu de culture comprend :
- les nutriments nécessaires à la croissance des bactéries *Yersinia enterocolitica* pathogènes, et
- au moins un agent chromogène substrat d'une acétyl-glucosaminidase.

3. Procédé selon la revendication 1 ou utilisation selon la revendication 2 **caractérisés en ce que** ledit,agent chromogène substrat d'une acétyl-glucosaminidase est une indoxyl-beta-glucosaminide.

4. Procédé selon l'une quelconque des revendications 1 ou 3, ou utilisation selon l'une quelconque des revendications 2 ou 3, **caractérisés en ce que** l'indoxyl-beta-glucosaminide est choisie dans le groupe constitué par le 5-bromo-6-chloro-3-indoxyl-N-acétyl-beta-D-glucosaminide et le 6-chloro-3-indolyl-N-acétyl-beta-D-glucosaminide et 6-fluoro-indoxyl-N-acétyl-beta-D-glucosaminide, de préférence est le 5-bromo-6-chloro-3-indoxyl-N-acétyl-beta-D-glucosaminide.

5. Procédé selon l'une quelconque des revendications 1, 3, ou 4, ou utilisation selon l'une quelconque des revendications 2-4, **caractérisés en ce que** le milieu comprend en outre un agent chromogène substrat de la beta-glucosidase.

6. Procédé ou utilisation selon la revendication 5, **caractérisés en ce que** ledit agent chromogène substrat de la beta-glucosidase est le 5-Bromo-4-chloro-3-indolyl-beta-D-glucoside.

7. Procédé selon l'une quelconque des revendications 1 et 3-6, ou utilisation selon l'une quelconque des revendications 2-6, **caractérisés en ce que** ledit milieu de culture est un milieu de culture gélosé.

8. Procédé selon l'une quelconque des revendications 1 et 3-7, ou utilisation selon l'une quelconque des revendications 2-7, **caractérisés en ce que** ledit milieu de culture comprend ledit agent chromogène substrat d'une acétyl-glucosaminidase à une concentration comprise entre 0,01 à 0,5 g/l, préférentiellement de 0,05 à 0,2 g/l.

9. Procédé ou utilisation selon l'une quelconque des revendications 5 à 8, **caractérisés en ce que** ledit milieu de culture comprend ledit agent chromogène substrat de la beta-glucosidase à une concentration comprise entre 0,01 à 0,5 g/l, préférentiellement de 0,05 à 0,2 g/l.

10. Procédé selon l'une quelconque des revendications 1 et 3-8, ou utilisation selon l'une quelconque des revendications 2-8, **caractérisés en ce que** le milieu comprend en outre du 5-chloro-2-(2,4-dichlorophenoxy)phenol.

## Patentansprüche

1. Verfahren zum direkten Nachweis von pathogenen *Yersinia enterocolitica* Bakterien in einer Probe, das folgende aufeinanderfolgende Etappen umfasst:
a) die Inokulation, anhand der genannten Probe, eines Kulturmediums bestehend aus:
- den für das Wachstum der pathogenen Bakterien *Yersinia enterocolitica* erforderlichen Nährstoffe, und
- mindestens ein chromogenes Substrat einer Acetylglucosaminidase
b)die Inkubation des genannten Kulturmediums unter Bedingungen, die die Vermehrung der pathogenen Bakterien *Yersinia enterocolitica* ermöglichen, und
c) den Nachweis der im Kulturmedium gebildeten Bakterienkolonien, die den pathogenen Bakterien *Yersinia enterocolitica* entsprechen.

2. Verwendung eines Kulturmediums für den direkten Nachweis und die Differenzierung von pathogenen *Yersinia enterocolitica* Bakterien, **dadurch gekennzeichnet, dass** das genannte Kulturmedium aus:
- den erforderlichen Nährstoffen für die Vermehrung der pathogenen *Yersinia enterocolitica* Bakterien, und
- mindestens einem chromogenen Substrat einer Acetylglucosaminidase besteht.

3. Verfahren nach Patentanspruch 1 oder Verwendung nach Patentanspruch 2, **dadurch gekennzeichnet, dass** das genannte chromogene Substrat einer Acetylglucosaminidase eine Indoxyl-Beta-Glucosaminide ist.

4. Verfahren nach einem der Patentansprüche 1 oder 3, oder Verwendung nach einem der Patentansprüche 2 oder 3, **dadurch gekennzeichnet, dass** die Indoxyl-Beta-Glucosaminide aus der Gruppe der 5-Brom-6-Chlor-3-Indoxyl-N-Acetyl-Beta-D-Glucosaminide und der 6-Chlor-3-Indolyl-N-Acetyl-Beta-D-Glucosaminide und der 6-Fluor-Indoxyl-N-Acetyl-Beta-D-Glucosaminide, vorzugsweise aus der 5 -Brom- 6-Chlor- 3-Indoxyl-N-Acetyl-Beta-D- Glucosaminide gewählt wird.

5. Verfahren nach einer der Patenansprüche 1, 3 oder 4, oder Verwendung nach einer der Patenansprüche 2 bis 4, **dadurch gekennzeichnet, dass** das Kulturmedium darüber hinaus aus einem chromogenen Substrat der Beta- Glucosidase besteht.

6. Verfahren oder Verwendung nach Patentanspruch 5, **dadurch gekennzeichnet, dass** es sich bei dem genannten chromogenen Substrat der Beta-Glucosidase um das 5-Brom-4-Chlor- 3-Indolyl-Beta-D-Glucoside handelt.

7. Verfahren nach einer der Patentansprüche 1, und 3 bis 6, oder Verwendung nach einer der Patentansprüche 2 bis 6, **dadurch gekennzeichnet, dass** das genannte Kulturmedium ein Agarkulturmedium ist,

8. Verfahren nach einer der Patentansprüche 1, und 3 bis 7, oder Verwendung nach einer der Patentansprüche 2 bis 7, **dadurch gekennzeichnet, dass** das genannte Kulturmedium aus dem genannten chromogenen Substrat einer Acetyl-Glucosaminidase mit einer Konzentration zwischen 0,01 und 0,5 g/l, vorzugsweise mit einer Konzentration zwischen 0,05 und 0,2 g/l besteht.

9. Verfahren oder Verwendung nach einer der Patentansprüche 5 bis 8,**dadurch gekennzeichnet, dass** das genannte Kulturmedium aus dem genannten chromogenen Substrat der Beta-Glucosidase mit einer Konzentration zwischen 0,01 und 0,5 g/l, vorzugsweise zwischen 0,05 und 0,2 g/l besteht.

10. Verfahren nach einer der Patentansprüche 1, und 3 bis 8, oder Verwendung nach einer der Patentansprüche 2 bis 8, **dadurch gekennzeichnet, dass** das Kulturmedium darüber hin aus 5-Chlor-2-(2,4-Dichlorphenoxy)Phenol besteht.

## Claims

1. Direct detection method for pathogenic *Yersinia enterocolitica* bacteria in a sample comprising the successive steps of:
a) inoculation with said sample of a culture medium comprising:
- nutrients necessary for growth of said pathogenic *Yersinia enterocolitica* bacteria, and
- at least one chromogenic agent substrate of an acetyl-glucosaminidase
b) incubation of said culture medium under conditions conducive to the growth of pathogenic *Yersinia enterocolitica* bacteria, and
c) detection of colonies formed on said culture medium corresponding to pathogenic *Yersinia enterocolitica* bacteria.

2. Use of a culture medium for direct detection and differentiation of pathogenic *Yersinia enterocolitica* bacteria, **characterised in that** said culture medium comprises:
- nutrients necessary for growth of pathogenic *Yersinia enterocolitica* bacteria, and
- at least one chromogenic agent substrate of an acetyl-glucosaminidase

3. Method according to claim 1 or use according to claim 2, **characterised in that** said chromogenic agent substrate of an acetyl-glucosaminidase is an indoxyl-beta-glucosaminide.

4. Method according to any one of claims 1 or 3, or use according to any one of claims 2 or 3, **characterised in that** the indoxyl-beta-glucosaminide is chosen from the group composed of 5-bromo-6-chloro-3-indoxyl-N-acetyl-beta-D-glucosaminide and 6-chloro-3-indolyl-N-acetyl-beta-D-glucosaminide and 6-fluoro-indoxyl-N-acetyl-beta-D-glucosaminide, preferably 5-bromo-6-chloro-3-indoxyl-N-acetyl-beta-D-glucosaminide.

5. Method according to any one of claims 1, 3 or 4, or use according to any one of claims 2-4, **characterised in that** the medium further comprises a chromogenic agent substrate of beta-glucosidase.

6. Method or use according to claim 5 **characterised in that** said chromogenic agent substrate of beta-glucosidase is 5-bromo-4-chloro-3-indolyl-beta-D-glucoside.

7. Method according to any one of claims 1 and 3-6, or use according to any of claims 2-6, **characterised in that** said culture medium is an agar-based culture medium.

8. Method according to any one of claims 1 and 3-7, or use according to any one of claims 2-7, **characterised in that** said culture medium comprises said chromogenic agent substrate of acetyl-glucosaminidase at a concentration of between 0.01 and 0.5 g/l, preferably 0.05 and 0.2 g/l.

9. Method or use according to any one of claims 5 to 8, **characterised in that** said culture medium comprises said chromogenic agent substrate of beta-glucosidase at a concentration of between 0.01 and 0.5 g/l, preferably between 0.05 and 0.2 g/l.

10. Method according to any one of claims 1 and 3-8, or use according to any one of claims 2-8, **characterised in that** the medium further comprises 5-chloro-2-(2,4-dichlorophenoxy) phenol.
